(19) Europäisches Patentamt
European Patent Office
Office européen des brevets

(11) Numéro de publication: **0 251 852 B1**

(12) # FASCICULE DE BREVET EUROPEEN

(45) Date de publication de fascicule du brevet: **20.03.91**

(51) Int. Cl.[5]: **C07C 227/40**

(21) Numéro de dépôt: **87401322.0**

(22) Date de dépôt: **12.06.87**

(54) **Procédé de séparation d'aminoacides.**

(30) Priorité: **25.06.86 FR 8609410**

(43) Date de publication de la demande:
**07.01.88 Bulletin 88/01**

(45) Mention de la délivrance du brevet:
**20.03.91 Bulletin 91/12**

(84) Etats contractants désignés:
**AT BE CH DE ES FR GB GR IT LI LU NL SE**

(56) Documents cités:
**US-A- 2 681 927**
**US-A- 2 894 954**

**CHEMICAL ABSTRACTS, vol. 70, no. 13, 31 mars 1969, page 427, résumé no. 58275e, Columbus, Ohio, US; & JP-A- 68 22 085**

**CHEMICAL ABSTRACTS, vol. 80, no. 18, 6 mai 1974, page 261, résumé no. 99915p, Columbus, Ohio, US; V.K. RYKOV et al.: "Liquid ion-exchange extraction of methionine from waste waters"**

(73) Titulaire: **RHONE-POULENC CHIMIE**
**25, quai Paul Doumer**
**F-92408 Courbevoie Cédex(FR)**

(72) Inventeur: **Bitar, Marie-Christine**
**42, rue Marius Aufan**
**F-92300 Levallois Perret(FR)**
Inventeur: **Sabot, Jean-Louis**
**3. Avenue Pascal**
**F-78600 Maisons Lafitte(FR)**
Inventeur: **Aviron-Viollet, Paul**
**25bis, Chemin de la Citadelle**
**F-69230 Saint Genis Laval(FR)**

(74) Mandataire: **Fabre, Madeleine-France et al**
**RHONE-POULENC INTERSERVICES Service Brevets Chimie 25, quai Paul Doumer**
**F-92408 Courbevoie Cédex(FR)**

**EP 0 251 852 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne un procédé de séparation d'aminoacides à partir d'un mélange d'au moins deux aminoacides. Elle concerne plus particulièrement un procédé de préparation d'aminoacides à partir d'hydrolysates de protéines.

Il existe diverses voies d'accès aux acides aminés dont les plus connues sont les voies de synthèse chimique ou biochimique. Ces voies conduisent généralement à un aminoacide unique en solution dans le milieu de préparation. L'extraction de cet aminoacide unique et sa purification sont généralement réalisables au niveau industriel.

Certains acides aminés sont aujourd'hui encore difficilement accessibles industriellement par voie chimique ou par fermentation.

Les acides aminés existent tous à l'état condensé à l'intérieur des protéines, constituants de tout être vivant végétal ou animal ; aussi on a cherché à hydrolyser ces protéines pour en extraire les divers aminoacides. A l'intérieur des matières protéiques, les acides aminés se trouvent alors mélangés en grand nombre de l'ordre de 20 environ.

Diverses méthodes ont été essayées pour chercher à séparer ces aminoacides. Il faut en effet noter que tous ces acides aminés ont des propriétés physicochimiques assez peu différentes et que dans la matière vivante, il se trouvent tous en présence, souvent sans constituant vraiment majoritaire ce qui augmente encore la difficulté de leur séparation.

Parmi les méthodes de séparation proposées dans la littérature, on a essayé : (1) la précipitation, mais l'efficacité de la séparation est médiocre et la nécessité de récupérer le réactif de précipitation complique le procédé ; ainsi le brevet US 2.681.927 décrit un procédé de séparation d'acides aminés dans une solution cétonique ou de dioxanne de phosphates acides d'alkyle que l'on précipite sélectivement ; (2) les procédés par échange d'ions qui sont difficiles à mettre en oeuvre du fait de la grande taille des colonnes, de la forte dilution de la solution et du mode de fonctionnement séquentiel de ces colonnes.

Il est aussi connu d'après le brevet américain US 2.894.954 de séparer sélectivement les aminoacides à l'aide d'une solution d'alcool aliphatique à partir de solutions acides aminés, les capacités d'extraction du solvant sont toujours faibles, cet inconvénient a été partiellement surmonté par l'adjonction d'hexylamine, mais même dans ce dernier cas, la sélectivité est trop faible.

Aucun des procédés de l'art antérieur ne permet de séparer sélectivement à partir de mélanges d'aminoacides, et à un coût avantageux inférieur au coût d'obtention par pure synthèse chimique ou biologique, les divers aminoacides naturels demandés par le marché que sont notamment l'arginine, la phénylalanine, le leucine, l'isoleucine, la valine et l'histidine.

On connaît également des procédés qui permettent de récupérer un ou plusieurs acides aminés d'un milieu les contenant. Parmi de tels procédés on peut citer ceux décrits dans Chemical Abstracts, Vol 70, N° 13, 31 mars 1969, page 427, résumé N° 58275e, Colombus, Ohio, US et dans Chemical Abstracts, vol 80, N° 18, 6 mai 1974, page 261, résumé N° 99915 p, Colombus Ohio US. Ces procédés utilisent certains acides organophosphoriques pour récupérer des acides aminés. Toutefois, ces procédés ne permettent pas une séparation sélective des acides aminés entre eux.

La présente invention a permis d'atteindre cet objectif et a pour objet un procédé de séparation d'acides aminés caractérisé en ce qu'on extrait à contre-courant sélectivement les acides aminés d'un mélange d'au moins deux aminoacides, épuisé en cystine, par un acide organo phosphoré, en solution dans au moins un alcool, une cétone, un éther et/ou au moins un hydrocarbure.

Les mélanges d'aminoacides, utilisés dans la présente invention peuvent être d'origine très diverses : synthèses biochimiques, chimiques, hydrolysats de protéines, bioconversions. Les protéines utilisées dans la présente invention peuvent elles aussi provenir de sources très diverses, kératine, agricole, animale, hémoglobine.

L'hydrolysat de protéines utilisé de préférence selon le procédé de l'invention est notamment un hydrolysat de divers déchets kératiniques tels que les cheveux, la laine, les plumes, les cornes, les sabots. L'hydrolyse est réalisée, à l'aide par exemple d'acides minéraux forts tels que les acides sulfuriques ou chlorhydrique, de bases fortes telles que la soude ou la potasse et d'enzymes, pendant des périodes variables pouvant durer notamment de 6 à 24 heures et à des températures elles aussi variables mais étant de préférence situées à environ 100° C notamment pour les hydrolyses chimiques. La solution aqueuse acide obtenue est concentrée si nécessaire, après ou avant l'élimination de la cystine, notamment par précipitation à un pH supérieur, à l'aide par exemple de soude.

Le mélange d'au moins deux acides aminés obtenu de préférence par hydrolyse de protéines contient au minimum 100 g/l d'aminoacides. Une concentration supérieure à 200 g/l est préférée.

Cette solution acide est mise en contact à contre-courant avec une solution organique extractante

composée d'un acide organophosphoré, d'un alcool aliphatique linéaire ou ramifié contenant de préférence 6 à 14 atomes de carbone et encore plus préférentiellement 8 à 10 atomes de carbone et/ou d'un hydrocarbure aliphatique.

L'acide organophosphoré est choisi parmi les acides dialkylphosphoriques, dialkylphosphoniques, dialkylphosphiniques, diarylphosphoriques, diarylphosphoniques, diarylphosphiniques. On préfère tout particulièrement utiliser l'acide diéthyl-2 hexylphosphorique.

Les acides dialkyl ou diarylphosphoriques répondent à la formule générale (I)

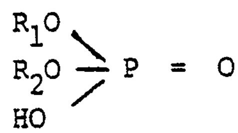

dans laquelle $R_1$ et $R_2$ représente des radicaux hydrocarbonés aromatiques et/ou aliphatiques contenant 1 à 18 atomes de carbone et dans lesquels au moins un groupe R comprend de 4 à 15 atomes de carbone.

Les acides dialkyl ou diarylphosphoniques répondent à la formule générale (II)

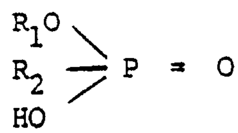

dans laquelle $R_1$ et $R_2$ ont la même signification que précédemment.

Les acides dialkyl ou diarylphosphiniques répondent à la formule générale (III)

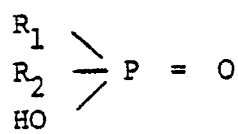

dans laquelle $R_1$ et $R_2$ ont la même signification que dans la formule (I)

Les alcools, cétones et éthers éventuellement mis en oeuvre sont choisis notamment parmi les composés comportant au moins 5 atomes de carbone.

On préfère mettre en oeuvre un alcool linéaire ou ramifié contenant 8 à 10 atomes de carbone et tout particulièrement l'éthyl-2-hexanol.

Les hydrocarbures éventuellement mis en oeuvre sont choisis parmi les diluants de 1' acide organophosphorè non miscibles à l'eau tels que les hydrocarbures aliphatiques linéaires ou ramifiés contenant au moins cinq atomes de carbone, les hydrocarbures aromatiques, les hydrocarbures halogénés. Des mélanges d'entre eux sont aussi utilisables tels que notamment des coupes pétrolières comme le kérosène ou de type Solvesso®.

On préfère utiliser le kérosène et encore plus préférentiellement un mélange alcool-kérosène.

Le solvant d' extraction particulièrement préféré pour la mise en oeuvre de l'invention présente la composition volumique suivante :

- acide diéthyl-2 herylphosphorique 40 - 80 %
- éthyl-2 hexanol 5 - 20 %
- kérosène 55 - 0 %

Les principaux aminoacides demandés par le marché que sont la valine, la leucine, l'isoleucine, la pnénylalanine, l'arginine et l'histidine sont notamment extraits de la solution d'hydrolysat de protéines épuisée en cystine par les techniques d'extraction suivantes.

- la pnénylalanine est extraite par un sel d'ammonium quaternaire à pH 10 à partir d'une solution d'acides aminés en milieu sulfurique ; parmi les sels d'ammonium quaternaire, on peut choisir les chlorures ou sulfates d'ammonium quaternaire répondant à la formule $R_3N^+ CH_3 CL^-$ ou $(R_3N^+ CH_3)_2 SO_4$ dans laquelle le radical R a de 8 à 10 atores de carbone ; ces produits sont couramment commercialisés sous les marques Adogen 464 et Aliquat 336; ils sont utilisés notamment en solution dans le Solvesso® à une concentration de 3 à 20 % en poids.
- l'arginine est séparée par une double extraction à l'aide des solvants d'extraction préalablement définis à pH 2,7 et 7.

- la leucine est extraite par les solvants d'extraction à pH3.
- l'isoleucine est séparée à partir d'une solution d'acides aminés épuisée en pnénylalanine à l'aide des solvants d'extraction à $pH^3$.

L'invention va être plus complètement décrite à l'aide des exemples suivants qui ne doivent pas être considérés comme limitatifs de l'invention.

EXEMPLE 1

On effectue la séparation des amino-acides à partir d'un hydrolysat de plumes épuisé en cystine qui présente à pH 2,7 la composition suivante :

| | |
|---|---|
| - Leucine | 21 g/l |
| - Isoleucine | 11,3 g/l |
| - Phénylalanine | 15 g/l |
| - Arginine | 24,5 g/l |
| - Valine | 17 g/l |
| - Serine | 39,5 g/l |
| - Threonine | 15 g/l |
| - Proline | 37,6 g/l |
| - Alanine | 15 g/l |
| - Acide glutamique | 33,8 g/l |
| - Acide aspartique | 24,5 g/l |
| - Glycine | 26,3 g/l |
| - Lysine | 5,6 g/l |
| - Méthionine | 1,9 g/l |
| - Histidine | 3,7 g/l |
| - NaCl | 150 g/l |

soit 291,7 g/l en amino-acides.
On met en contact cette solution avec la solution extractante composée :

| | |
|---|---|
| d'acide diéthyl-2 hexyl phosphorique | 75 % |
| éthyl-2 hexanol | 10 % |
| kérosène | 15 % |

Cette opération s'effectue selon le dispositif décrit à la figure 1 dans une batterie d'extraction liquide I fonctionnant à contre-courant et comprenant 9 étages théoriques. Le solvant alimente la batterie à l'étage I à un débit de 100 ml/h. La solution d'amino-acides à pH 2,7 est introduite dans la batterie à l'étage 5 à un débit de 100 ml/h. Le lavage sélectif est réalisé par introduction à l'étage 9 d'une solution de chlorure de sodium à 80 g/l dans l'acide chlorhydrique $10^{-3}$N, à un débit de 40 ml/h. Le pH d'extraction est régulé à pH 2,7 par ajouts de soude 10 N dans les mélangeurs des étages 2 et 5 aux débits de 10 ml/h.

La phase aqueuse épuisée B sortant de l'étage 1 contient :

| | | |
|---|---|---|
| - Arginine | 17,5 | g/l |
| - Histidine | 2,6 | g/l |
| - Lysine | 4 | g/l |
| - Acide glutamique | 24,1 | g/l |
| - Glycine | 18,8 | g/l |
| - Acide aspartique | 17,5 | g/l |
| - Serine | 28,2 | g/l |
| - Threonine | 10,7 | g/l |
| - Alanine | 10,7 | g/l |
| - Proline | 26,8 | g/l |
| - Valine | < 60 | ml/l |
| - Phénylalanine | < 50 | ml/l |
| - Méthionine | < 6 | mg/l |
| - Leucine | < 15 | mg/l |
| - Isoleucine | < 8 | mg/l |
| - NaCl | 100 | g/l |

ce qui correspond à un taux d'extraction supérieur à :

. 99,5 % pour la valine, méthionine et phénylalanine
. 99,9 % pour la leucine et l'isoleucine

Le solvant chargé sortant de la batterie 1 alimente une batterie 2 de trois étages dans laquelle on récupère les amino-acides extraits en solution aqueuse par mise en contact du solvant à contre courant avec une solution d'acide chlorhydrique 2 N au débit de 25 ml.h. La solution aqueuse A d'amino-acides obtenue a la composition suivante :

| | | |
|---|---|---|
| - Leucine | 84 | g/l |
| - Isoleucine | 45,2 | g/l |
| - Phénylalanine | 60 | l/l |
| - Valine | 68 | l/l |
| - Méthionine | 7,6 | g/l |
| autres amino-acides | < 2,4 | g/l |

La solution B d'amino-acides est amenée en continu à pH 7 par ajout de soude 10 N au débit de 10 ml.h. La solution obtenue se compose comme suit :

| | |
|---|---|
| - Arginine | 16,3 g/l |
| - Histidine | 2,5 g/l |
| - Lysine | 3,7 g/l |
| - Acide glutamique | 22,4 g/l |
| - Glycine | 17,5 g/l |
| - Acide aspartique | 16,3 g/l |
| - Serine | 26,3 g/l |
| - Threonine | 10 g/l |
| - Alanine | 10 g/l |
| - Proline | 25 g/l |
| - Autres amino-acides | < 0,2 g/l |
| - NaCl | 92 g/l |

Elle est mise en contact avec la solution extractante composée

| | |
|---|---|
| d'acide diéthyl-2 hexyl phosphorique | 75 % |
| éthyl-2 hexanol | 10 % |
| kérosène | 15 % |

Cette opération s'effectue selon le dispositif décrit à la figure 1 dans une batterie 3 d'extraction liquide-liquide fonctionnant à contre courant et comprenant 20 étages théoriques. Le solvant alimente la batterie à l'étage I au débit de 150 ml/h. La solution d'acides aminés est introduite à l'étage 7 au débit de 150 ml/l. Le lavage sélectif est effectué par une solution de chlorure de sodium à 80 g/l au débit de 80 ml/h. Le pH d'extraction est régulé à 7 par de la soude 10 N introduite dans les mélangeurs 3 et 7 aux débits de 5 ml/h.

La phase aqueuse épuisée F sortant de l'étage I contient plus de 99 % de chaque acide aminé, lysine, acides glutamique et aspartique, glycine, serine, thréonine, proline, alanine, histidine et autres amino-acides et moins de 150 mg/l d' arginine ce qui correspond à un taux d'extraction de l'arginine supérieur à 99 %. La solution F se compose comme suit :

| | |
|---|---|
| - Lysine | 2,4 g/l |
| - Acide glutamique | 14,6 g/l |
| - Glycine | 11,4 g/l |
| - Acide aspartique | 10,6 g/l |
| - Serine | 17,1 g/l |
| - Threonine | 6,5 g/l |
| - Alanine | 6,5 g/l |
| - Proline | 16,2 g/l |
| - Histidine | 1,6 g/l |
| - Autres amino-acides | ⩽ 0,12 g/l |
| - Arginine | ⩽ 0,15 g/l |
| - NaCl | 80 g/l |

Le solvant chargé en arginine sortant de la batterie 3 alimente une batterie 4 de trois étages dans laquelle on récupère l'arginine extraite en solution aqueuse par contact à contre courant avec une solution d'acide chlorhydrique 2 N au débit de 25 ml/h.

La phase aqueuse obtenue est une solution aqueuse acide d'arginine à 97,8 g/l contenant moins de 3 g/l d'autres acides aminés.

La phase aqueuse épuisée F est mise alors en contact avec une phase organique composée :

| | |
|---|---|
| d'acide diéthyl-2 hexyl phosphorique | 75 % |
| d'éthyl-2 hexanol | 10 % |
| de kérosène | 15 % |

Cette opération est réalisée selon le dispositif décrit à la figure 1 dans une batterie d' extraction liquide-liquide 12 fonctionnant à contre courant et comprenant 19 étages théoriques. La solution aqueuse F alimente la batterie à l'étage 10 au débit de 230 ml/h. Le solvant d'extraction est introduit à l'étage 1 au débit de 27 ml/h. Le lavage sélectif s'effectue par une solution d'acide chlorhydrique 0,15 N introduite à l'étage 19 au débit de 88,5 ml/h. De la soude 10 N est introduite à l'étage 1 au débit de 15 ml/h.

Dans ces conditions la phase aqueuse épuisée G sortant de l'étage 1 contient moins de 25 mg/l en histidine et plus de 99,9 % de chacun des autres amino-acides de la solution d' alimentation F. Ceci correspond à un rendement d'extraction de l'histidine supérieur à 99,9 %. Le solvant chargé en histidine sortant de l'étage 19 alimente une batterie d' extraction liquide-liquide 13 fonctionnant à contre-courant et comprenant 3 étages théoriques dans laquelle on récupère l' histidine extraite en phase aqueuse par contact avec une solution d'acide chlorhydrique 2N au débit de 10 ml/h. On obtient une phase aqueuse acide d'histidine à 37,5 g"l contenant moins de 5 mg/l d'autres amino-acides soit une pureté en histidine d'au moins 99,95 %.

L'extrait régénéré de la première séparation, noté A, est repris et neutralisé en continu par ajout de soude 10 N au débit de 15 ml/h.

La solution A se compose de :

| | | |
|---|---|---|
| - Leucine | 52,5 | g/l |
| - Isoleucine | 28,5 | g/l |
| - Phénylalanine | 37,5 | g/l |
| - Valine | 42,5 | g/l |
| - Méthionine | 4,8 | g/l |
| - Autres amino-acides | $\leqslant$ 1,5 | g/l |
| - NaCl | 80 | g/l |

on la met en contact avec une phase organique composée :

| | |
|---|---|
| d'acide diéthyl-2 hexyl phosphorique | 75 % |
| d'éthyl-2 hexanol | 10 % |
| de kérosène | 15 % |

Cette opération est réalisée selon le dispositif décrit à la figure 1 dans une batterie d'extraction liquide-liquide 5 fonctionnant à contre courant et comprenant 24 étages théoriques. Le solvant alimente la batterie à l'étage l à un débit de 170 ml/k. La solution d'acides aminés est introduite à l'étage 12 à un débit de 40 ml/h. Le lavage sélectif est réalisé par une solution d'acide chlorhydrique 1,25 N au débit de 55 ml/h. De la soude 10 N est introduite à l'étage 1 au débit de 10 ml/h. La phase aqueuse épuisée C sortant de l'étage 1 contient plus de 99,8 % de chaque acide aminé : valine, méthionine et autres amino-acides et moins de 10 mg/l de leucine, isoleucine et phénylalanine, ce qui correspond à un taux d'extraction supérieur à 99,9 % pour ces trois acides.

La solution C se compose de :

| | | |
|---|---|---|
| - Valine | 17,9 | g/l |
| - Méthionine | 2 | g/l |
| - Autres amino-acides | ⩽ 0,6 | g/l |
| - Leucine | < 10 | ml/l |
| - Isoleucine | < 10 | mg/l |
| - Phénylalanine | < 10 | mg/l |
| - NaCl | 33 | g/l |

Le solvant chargé sortant de la batterie 5 alimente une batterie 6 de 3 étages dans laquelle on récupère les amino-acides extraits en phase aqueuse par mise en contact à contre-courant du solvant avec de l'acide sulfurique 2 N au débit de 40 ml/h. La phase aqueuse obtenue D se compose de :

| | | |
|---|---|---|
| - Leucine | 52,5 | g/l |
| - Isoleucine | 28,5 | g/l |
| - Phénylalanine | 37,5 | g/l |
| - Autres amino-acides | ⩽ 0,2 | g/l |

Cette solution est neutralisée par de la soude 10 N en continu au débit de 8 ml/h. Elle présente alors la composition suivante :

| | | |
|---|---|---|
| - Leucine | 42,7 | g/l |
| - Isoleucine | 23,7 | g/l |
| - Phénylalanine | 31,7 | g/l |
| - Autres amino-acides | ⩽ 0,2 | g/l |
| - $Na_2SO_4$ | 142 | g/l |

La phénylalanine est extraite de cette solution par contact en continu et à contre-courant avec la phase organique composée

d'Aliquat® 336 15% sous forme de chlorure

Solvesso® 150 85 %

Ce contact est réalisé selon le dispositif de la figure 1, dans une batterie d'extraction liquide liquide 7 constituée de 16 étages théoriques. Le solvant alimente la batterie à l'étage 1, au débit de 140 ml/h. La solution d'amino-acides est introduite à l'étage 8, au débit de 48 ml/h. Le lavage sélectif est effectué par une solution d'acide sulfurique 0,7 N au débit de 26 ml/h introduite à l'étage 16.

De la soude 10 N est introduite à l'étage 1 au débit de 3 ml/h. Dans ces conditions, la phénylalanine extraite se trouve dans le solvant à une pureté de 99,9 % pour un rendement d'extraction égal à 99,7 %.

La phase aqueuse épuisée E sortant de l'étage 1 se compose de :

| | |
|---|---|
| - Leucine | 28,4 g/l |
| - Isoleucine | 15,4 g/l |
| - Autres amino-acides | ≤ 0,1 g/l |
| - $Na_2SO_4$ | 92 g/l |

Le solvant chargé en phénylalanine sortant de la batterie 7 alimente une batterie 8 de 3 étages dans laquelle on récupère la phénylalanine extraite en phase aqueuse par mise en contact à contre-courant du solvant avec une solution d'acide sulfurique 2 N au débit de 24 ml/h. On obtient une solution aqueuse acide de phénylalanine à 87,5 g"l et contenant moins de 0,1 % d'autres amino-acides.

La solution épuisée E contenant principalement la leucine et l' isoleucine alimente une batterie d' extraction 9 liquide-liquide fonctionnant à contre-courant et comportant 21 étages théoriques. Le solvant constitué :

| | |
|---|---|
| d'acide diéthyl-2 hexyl phosphorique | 75 % |
| éthyl-2 hexanol | 10 % |
| kérosène | 15 % |

alimente la batterie à l'étage 1 au débit de 90 ml/h.

La solution d'acides aminés est introduite à l'étage 12 au débit de 74 ml/h. Le lavage sélectif est effectué par une solution d'acide sulfurique 0,34 N introduite à l'étage 21 au débit de 130 ml h. De la soude 10 N est introduite à l'étage 1 au débit de 4 ml h. Dans ces conditions, la phase aqueuse épuisée est une solution aqueuse d' isoleucine à 5,6 g/l et de pureté supérieure à 98,8 %.

Le solvant chargé en leucine est introduit dans une batterie d' extraction liquide-liquide 10 fonctionnant à contre-courant comportant 3 étages théoriques. Le leucine extraite est remise en phase aqueuse par contact du solvant chargé avec une solution d' acide chlorhydrique 2 N au débit de 20 ml/h. On obtient une solution aqueuse acide de leucine à 105 g/l et de pureté d'au moins 99,8 %.

EXEMPLE 2

L'extrait régénéré de la lère séparation de l'exemple 1, noté A, est repris et neutralisé en continu par ajout de soude 10N au débit de 15 ml/h.

La solution se compose de :

| | |
|---|---|
| - Valine | 42,50 g/l |
| - Leucine | 52,50 g/l |
| - Isoleucine | 28,50 g/l |
| - Phénylalanine | 37,50 g/l |
| - Méthionine | 4,75 g/l |
| - Tyrosine | 4,75 g/l |
| - Autres acides aminés | ≤ 1,50 g/l |
| - $Na_2SO_4$ | 142 g/l |

La phénylalanine est extraite de cette solution par contact en continu à contre-courant avec une phase organique constituée d'Aliquat® 336 sous forme chlorure à 15 % en volume dans le kérosène.

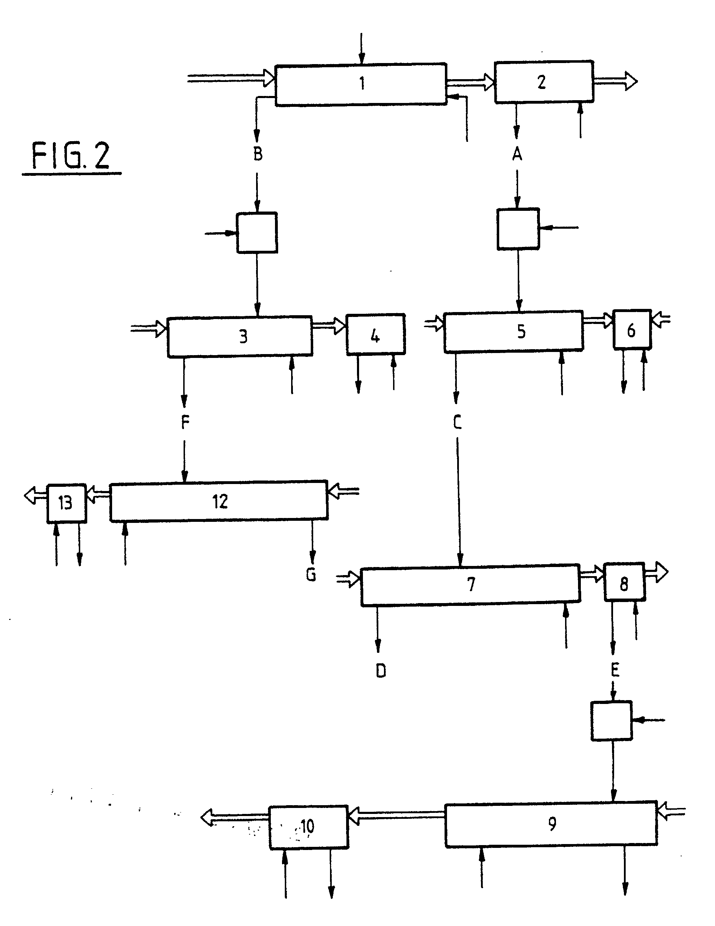

Cette opération est réalisée selon le dispositif de la figure 2 dans une batterie d' extraction liquide-liquide 5 fonctionnant à contre-courant et comprenant 16 étages théoriques. Le solvant alimente la batterie

à l'étage 1 au débit du 117 ml/h. La solution d'amino-acides est introduite à l'étage 8 au débit de 40 ml/h. Le lavage sélectif s'effectue par une solution d'acide sulfurique 0,85 N au débit de 22 ml/h introduite à l'étage 16. De la soude 10 N est introduite à l'étage 1 au débit de 2,5 ml/h.

Dans ces conditions la phénylalanine se trouve dans le solvant à une pureté de 99,9 % et son rendement d'extraction est égal à 99,7 %. Le solvant chargé en phénylalanine sortant de la batterie alimente une batterie 6 de 3 étages dans laquelle on récupère la phénylalanine extraite en phase aqueuse par mise en contact à contre-courant du solvant avec une solution d'acide sulfurique 2N au débit de 20 ml/h. On obtient une solution aqueuse acide de phénylalanine à 75 g/l et contenant moins de 0,1 % d'autres amino-acides.

La solution épuisée C sortant de l'étage 1 se compose de :

| | | |
|---|---|---|
| - Leucine | 33,9 | g/1 |
| - Isoleucine | 18,4 | g/1 |
| - Valine | 27,4 | g/1 |
| - Méthionine | 3,1 | g/1 |
| - Tyrosine | 3,1 | g/1 |
| - $Na_2SO_4$ | 142 | g/1 |
| - Autres acides aminés | $\leqslant$ 1 | g/1 |

Elle est mise en contact avec une phase organique composée :

| | |
|---|---|
| d'acide diéthyl-2 hexyl phosphorique | 75 % |
| d'éthyl-2 hexanol | 10 % |
| de kérosène | 15 % |

Cette opération est réalisée selon le dispositif décrit à la figure 2, dans une batterie d'extraction liquide-liquide 7 fonctionnant à contre-courant et comprenant 21 étages théoriques. Le solvant alimente la batterie à l'étage 1 au débit de 218 ml/h. La solution d'amino-acides est introduite à l'étage 11 au débit de 62 ml/h. Le lavage sélectif est réalisé par une solution d'acide sulfurique 0,7 N au débit de 156 ml/h. De la soude 10 N est introduite à l'étage 1 au débit de 13,5 ml/h.

La phase aqueuse épuisée D sortant de l'étage 1 contient plus de 99,8 % de chaque amino acide valine, méthionine, tyrosine et autres amino acides et moins de 10 mg/l de leucine et isoleucine, ce qui correspond à un taux d'extraction de ces deux amino acides de 99,9 %. La solution D se compose de :

| | | |
|---|---|---|
| - Leucine | $\leqslant$ 10 | mg/1 |
| - Isoleucine | $\leqslant$ 10 | mg/1 |
| - Valine | 7,8 | g/1 |
| - Méthionine | 0,9 | g/1 |
| - Tyrosine | 0,87 | g/1 |
| - Autres acides aminés | $\leqslant$ 0,3 | g/1 |
| - $Na_2SO_4$ | 40 | g/1 |

Le solvant chargé sortant de la batterie 7 alimente une batterie 8 de 3 étages dans laquelle on récupère les amino acides extraits, principalement leucine et isoleucine, par mise en contact du solvant avec une solution d'acide chlorhydrique 2N. Après neutralisation à la soude 10N, cette solution E se compose de :

- Leucine 40 g/l
- Isoleucine 28,50 g/l
- Autres acides aminés < 0,1 g/l

Elle alimente une batterie d'extraction 9 fonctionnant à contre courant et comportant 21 étages théoriques. Le solvant composé :

| | |
|---|---|
| d'acide diéthyl-2 hexyl phosphorique | 75 % |
| d'éthyl-2 hexanol | 10 % |
| de kérosène | 15 % |

alimente la batterie à l'étage 1 au débit de 18 ml/l la solution d'acides aminés est introduite à l'étage 12 au débit de 40 ml/h. Le lavage sélectif est effectué par de l'acide chlorhydrique 0,5 N au débit de 70 ml/h. De la soude 10 N est introduite à l'étage 1 au débit de 1,2 ml/h. Dans ces conditions la phase aqueuse épuisée est une solution d'isoleucine à 10,4 g/l et de pureté supérieure à 98,8 %

Le solvant chargé contenant la leucine est contacté à contre courant avec une solution d'acide chlorhydrique 2N au débit de 10 ml/h dans une batterie 10 de 3 étages théoriques. On obtient une solution aqueuse acide de leucine à 160 g/l et de pureté d'au moins 99,8 %.

EXEMPLE 3

5 ml d'une solution 0,093 molaire en L phénylalanine et 0,052 molaire en acide aspartique provenant d'une réaction de transamination réalisée à pH 7,5 sont amenés à pH 2,9 par addition d'acide chlorhydrique concentré. A cette solution sont ajoutés 5 ml d'un mélange d'acide bis ( éthyl-2 hexyl ) phosphorique, d' éthyl-2 hexanol et de kérosène ayant la composition volumique suivante :

| | |
|---|---|
| - acide bis (éthyl-2 hexyl) phosphorique | 75 % |
| - éthyl-2 hexanol | 10 % |
| - kérosène | 15 % |

Le mélange est agité et les deux phases sont séparées, la teneur en aminoacide des deux phases est déterminée. 94,2 % de la L phénylalanine présente dans la solution initiale passe dans la phase organique alors que 95 % de 1' acide L aspartique reste dans la phase aqueuse.

EXEMPLE 4

A 450 ml de la même solution qu'à l'exemple 1, amenée à pH 2,9 sont ajoutés 450 ml d'un mélange d'acide bis (éthyl-2 hexyl) phosphorique, d' éthyl-2 hexanol et de kérosène ayant la composition volumique suivante :

| | |
|---|---|
| - acide bis (éthyl-2 hexyl) phosphorique | 50 % |
| - éthyl-2 hexanol | 6,7 % |
| - kérosène | 43,3 % |

Le mélange est agité puis après décantation les deux phases sont séparées. La phase organique est contre extraite par un volume équivalent d' acide chlorhydrique 2 N. Le dosage de la L phénylalanine dans les phases aqueuses de la première extraction indique que 87 % de la L phénylalanine a été extraite.

EXEMPLE 5

5 ml d'une solution aqueuse 0,05 molaire en L tryptophane, 0,05 molaire en L serine et 0,083 molaire en glycine sont amenés à pH 3 par addition d'HCl concentré. A cette solution sont ajoutés 5 ml d'un mélange d'acide bis (éthyl-2 hexyl) phosphorique, d'éthyl-2 hexanol et de kérosène ayant une composition volumique suivante :

- acide bis (éthyl-2 hexyl) phosphorique 75 %
- éthyl-2 hexanol 10 %
- kérosène 15 %

Le mélange est agité puis après décantation les deux phases sont séparées. L'analyse de la phase aqueuse montre que 96 % du L tryptophane ont été extraits dans la phase organique, la L serine et la glycine se retrouvent à plus de 95 % dans cette phase aqueuse.

EXEMPLE 6

5 ml d'une solution aqueuse 0,05 molaire en L tryptophane, 0,3 molaire en L sérine et 0,75 molaire en glycine sont amenés à pH 3 par de l'acide chlorhydrique concentré. A cette solution sont ajoutés 5 ml d'un mélange extractant de même composition que celle donnée dans l'exemple 5.

Après agitation et décantation l'analyse de la phase aqueuse montre que 97 % du L tryptophane sont passés dans la phase organique, et moins de 5 % de L sérine et de glycine ont été extraites.

**Revendications**

1. Procédé de séparation d'acides aminés caractérisé en ce qu'on extrait à contre courant sélectivement les acides aminés d'un mélange d'au moins deux acides aminés, épuisé en cystine, par un acide organophosphoré en solution dans au moins un alcool, une cétone, un éther et/ou au moins un hydrocarbure.

2. Procédé selon la revendication 1 caractérisé en ce que le mélange d'acides aminés est un hydrolysat de protéines naturelles

3. Procédé selon la revendication 1, caractérisé en ce que le mélange d'acides aminés issu de l' hydrolysat de protéines contient au moins 100 g/l d'acides aminés.

4. Procédé selon la revendication 3 , caractérisé en ce que le mélange d'acides aminés contient au moins 200 g/l d'acides aminés

5. procédé selon la revendication 1, caractérisé en ce qu'on extrait sélectivement les acides aminés par une solution organique extractante composée d'un acide organophosphoré d'au moins un alcool aliphatique linéaire ou ramifié contenant 6 à 14 atomes de carbone et/ou d'au moins un hydrocarbure aliphatique.

6. Procédé selon la revendication 5, caractérisé en ce que l'acide organophosphoré est choisi parmi les acides dialkylphosphoriques, dialkylphosphoniques, dialkylphosphiniques, diarylphosphoriques, diarylphosphoniques, diarylphosphiniques.

7. Procédé selon la revendication 6, caractérisé en ce que l'acide organophosphoré est l'acide diéthyl-2 hexylphosphorique.

8. Procédé selon la revendication 5, caractérisé en ce que l'alcool aliphatique linéaire ou ramifié contient 8 à 10 atomes de carbone.

9. Procédé selon la revendication 8, caractérisé en ce que l'alcool aliphatique est l' éthyl-2 hexanol.

10. procédé selon la revendication 1 caractérisé en ce que parmi les hydrocarbures aliphatiques on choisit le kérosène.

11. Procédé selon la revendication 5, caractérisé en ce que la solution organique extractante a la composition volumique suivante :

| | | |
|---|---|---|
| acide diéthyl-2 hexylphosphorique | : | 40 - 80 % |
| éthyl-2 hexanol | : | 5 - 20 % |
| kérosène | : | 55 - 0 % |

## Claims

1. Process for the separation of amino acids, characterized in that the amino acids are selectively extracted countercurrently from a cystine-depleted mixture of at least two amino acids with an organophosphorus acid dissolved in at least one alcohol, a ketone, an ether and/or at least one hydrocarbon.

2. Process according to Claim 1 characterized in that the amino acid mixture is a hydrolysate of natural proteins.

3. Process according to Claim 1, characterized in that the amino acid mixture derived from the protein hydrolysate contains at least 100 g/l of amino acids.

4. Process according to Claim 3, characterized in that the amino acid mixture contains at least 200 g/l of amino acids.

5. Process according to Claim 1, characterized in that the amino acids are selectively extracted with an organic extractant solution consisting of an organophosphorus acid, at least one straight-chain or branched aliphatic alcohol containing 6 to 14 carbon atoms and/or at least one aliphatic hydrocarbon.

6. Process according to Claim 5, characterized in that the organophosphorus acid is chosen from amongst dialkylphosphoric, dialkylphosphonic, dialkylphosphinic, diarylphosphoric, diarylphosphonic and diarylphosphinic acids.

7. Process according to Claim 6, characterized in that the organophosphorus acid is 2-diethylhexylphosphoric acid.

8. Process according to Claim 5, characterized in that the straight-chain or branched aliphatic alcohol contains 8 to 10 carbon atoms.

9. Process according to Claim 8, characterized in that the aliphatic alcohol is 2-ethylhexanol.

10. Process according to Claim 1, characterized in that among the aliphatic hydrocarbons, kerosene is chosen.

11. Process according to Claim 5, characterized in that the organic extractant solution has the following composition by volume:

| | |
|---|---|
| 2-diethylhexylphosphoric acid: | 40 - 80% |
| 2-ethylhexanol: | 5 - 20% |
| kerosene: | 55 - 0% |

## Ansprüche

1. Verfahren zur Trennung von Aminosäuren, dadurch gekennzeichnet, daß man selektiv im Gegenstrom die Aminosäuren aus einem von Cystin befreiten Gemisch von wenigstens zwei Aminosäuren durch eine wenigstens in einem Alkohol, einem Keton, einem Ether und/oder wenigstens einem Kohlenwasserstoff gelöste, organische phosphorhaltige Säure extrahiert.

2. Verfahren nach Anspruch 1, dadurch gekennzeichnet, daß das Aminosäuregemisch ein Hydrolysat natürlicher Proteine ist.

3. Verfahren nach Anspruch 1, dadurch gekennzeichnet, daß das aus dem Proteinhydrolysat stammende Aminosäuregemisch wenigstens 100 g/l Aminosäuren enthält.

4. Verfahren nach Anspruch 3, dadurch gekennzeichnet, daß das Aminosäuregemisch wenigstens 200 g/l Aminosäuren enthält.

5. Verfahren nach Anspruch 1, dadurch gekennzeichnet, daß man die Aminosäuren durch eine organische Extraktionslösung selektiv extrahiert, die aus einer organischen phosphorhaltigen Säure, wenigstens einem 6 bis 14 Kohlenstoffatome enthaltenden geradkettigen oder verzweigten aliphatischen Alkohol und/oder wenigstens einem aliphatischen Kohlenwasserstoff besteht.

6. Verfahren nach Anspruch 5, dadurch gekennzeichnet, daß die organische phosphorhaltige Säure aus den Dialkylphosphor-, Dialkylphosphon-, Dialkylphosphin-, Diarylphosphor-, Diarylphosphon- und Diarylphosphinsäuren ausgewählt ist.

7. Verfahren nach Anspruch 6, dadurch gekennzeichnet, daß die organische phosphorhaltige Säure 2-Diethylhexylphosphorsäure ist.

8. Verfahren nach Anspruch 5, dadurch gekennzeichnet, daß der geradkettige oder verzweigte aliphatische Alkohol 8 bis 10 Kohlenstoffatome enthält.

9. Verfahren nach Anspruch 8, dadurch gekennzeichnet, daß der aliphatische Alkohol 2-Ethylhexanol ist.

10. Verfahren nach Anspruch 1, dadurch gekennzeichnet, daß man aus den aliphatischen Kohlenwasserstoffen Kerosin auswählt.

11. Verfahren nach Anspruch 5, dadurch gekennzeichnet, daß die organische Extraktionslösung die folgende Zusammensetzung in Volumenprozent hat:

| | | |
|---|---|---|
| 2-Diethylhexylphosphorsäure | : | 40 - 80 % |
| 2-Ethylhexanol | : | 5 - 20 % |
| Kerosin | : | 55 - 0 % |

FIG. 1

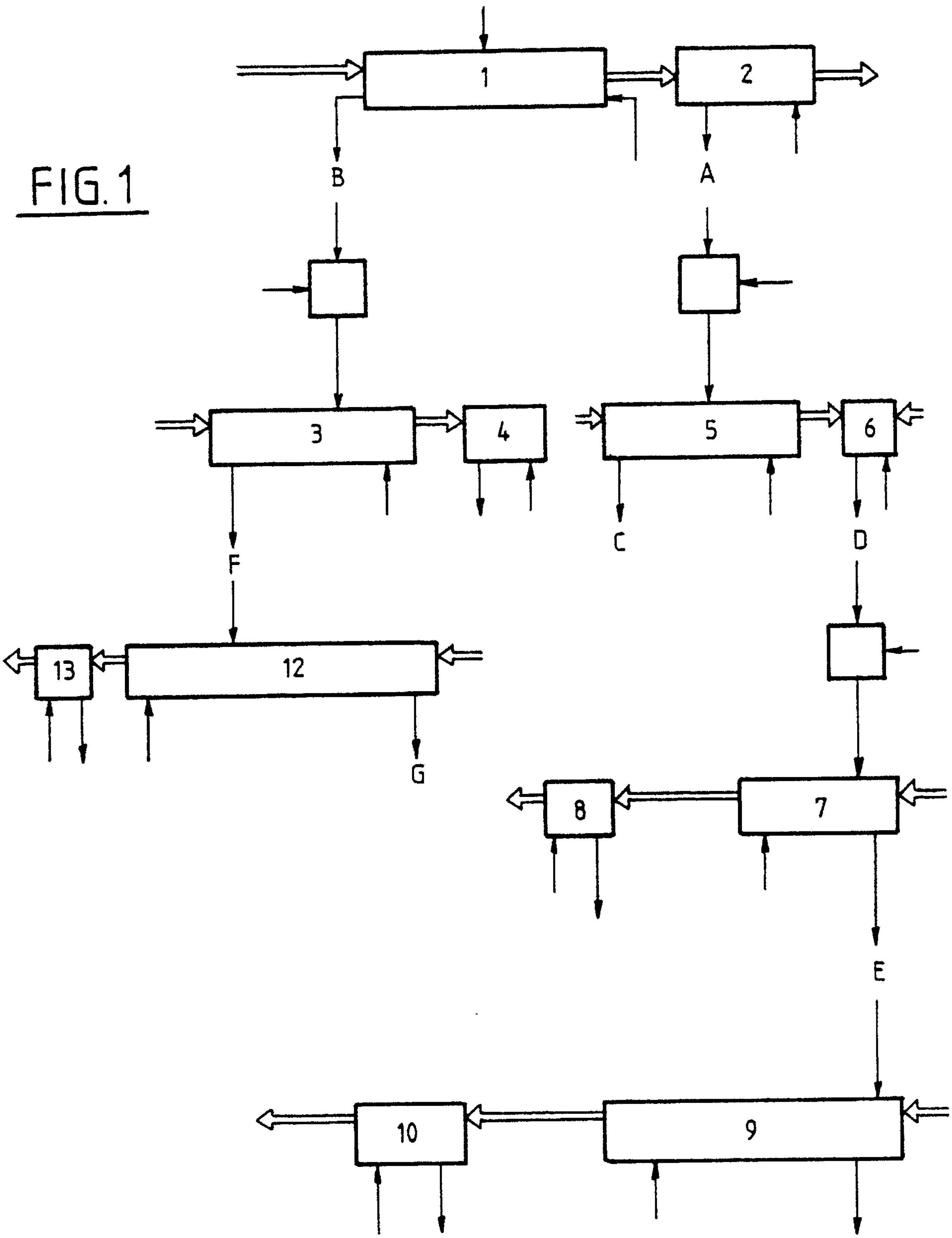

FIG. 2